# EUROPEAN PATENT SPECIFICATION

(11) **EP 1 561 034 B1**
(45) Date of publication and mention of the grant of the patent: **21.12.2011**
(21) Application number: 03779834.5
(22) Date of filing: 03.11.2003
(51) Int. Cl.: F04B 43/12

(54) **PERISTALTIC PUMP**
PERISTALTISCHE PUMPE
POMPE PERISTALTIQUE

(30) Priority: 13.11.2002 DE 20217483 U
(43) Date of publication of application: 10.08.2005
(73) Proprietor: ECOLAB INC., St. Paul, MN 55102-1390 (US)
(72) Inventor: HUBER, Wolfgang, 83413 Friedolfing (DE); RUTZ, Klaus, 83377 Vachendorf (DE); HEIDUCZEK, Ralf, 83483 Bischofswiesen (DE)
(74) Representative: Gesthuysen, von Rohr & Eggert
(86) International application number: PCT/EP2003/012214
(87) International publication number: WO 2004/044425

(56) References cited:
- EP-A- 0 786 596
- GB-A- 727 001
- US-A- 4 256 442

## Description

The invention relates to a peristaltic pump, in particular for use in dishwashers for the metering of a liquid or pasty cleaning agent, with the features of the introductory part of claim 1.

In a prior art solution of document DE-A1-39 43 430 a squeeze hose bend is accommodated in a plastics material yoke which forms the curved contact wall for a squeeze hose in the use position, wherein the curved contact wall is detented with a squeeze hose, which is detented in place, after being laid around the squeeze hose rollers at the pump body. The fastening of the squeeze wall in a plane, perpendicular to the squeeze roller axis, is also shown in DE-B-26 57 908 or DD-157 620.

Peristaltic pumps of other construction are known from DE-A1-33 26 766 or DE-T2-696 16 336 or from DE-U-71 47 400, to give only a few examples.

In the closest prior art of document US-A-4,256,442 the peristaltic pump comprises a base element and positioned on the base element a rotating squeeze roller carrier carrying squeeze rollers. A squeeze hose is partly looping around the squeeze rollers. A curved contact wall for the squeeze hose is provided along with a pivot lever for operating the curved contact wall.

This system comprises a four-bar linkage arrangement which automatically retains the curved contact wall in either its fully open or fully closed position as a result of an over-center type locking construction. So in a working position of the peristaltic pump the curved contact wall is positioned opposite to the effective surface of the squeeze rollers and presses the squeeze hose against the squeeze rollers, By outward pivoting movement of the pivot lever the curved contact wall is movable from the working position into an open position with the curved contact wall removed form the squeeze rollers. In that open position the squeeze hose can be removed from the peristaltic pump.

In above-mentioned construction of a peristaltic pump the squeeze hose bend as such has to be handled. Handling of the squeeze hose bend as such is at best tedious.

The object of the invention is to create a solution by which a simple exchange of the squeeze hose bend is made possible with a robust construction of all elements.

According to the invention above mentioned object is met with a peristaltic pump comprising the features of the characterizing part of claim 1 in combination with its features in the introductory part.

Preferred embodiments of the invention can be obtained form the sub-claims.

As in the prior art construction, because of the design of the curved contact wall movable by way of a pivot lever an extremely simple squeeze hose positioning is possible. The pivot lever is opened, wherein the curved contact wall is removed from the hose, the hose can be removed or exchanged. The pivot lever is closed and the curved contact wall and all other pump elements are disposed in the working position.

The curved contact wall forms a part of a pump housing cover on which the pivot lever is positioned to be outwardly pivotable for displacement of the pump housing cover. It is achieved by this design that on closing the pump housing cover the curved contact wall, which is integrally formed therewith, is simultaneously displaced into the working position.

The squeeze hose bend is provided on a squeeze hose carrier. In the locking setting of the pump head the squeeze hose carrier with the squeeze hose bend is positioned at the end wall of the base element. The squeeze hose carrier together with the squeeze hose bend is freed to be withdrawn from the pump head of the peristaltic pump in the unlocked setting thereof.

In a preferred embodiment of the invention support detents for receiving pivot cams of the setting lever are provided at the base element. In that case it can be advantageous in a further embodiment if counter rails or grooves for receiving slide rails at the pump housing cover are provided on the base element.

It can be advantageous that the end wall with a detent tongue as well as with two substantially U-shaped recesses for passage of the squeeze hose is provided at the housing base. In a further embodiment it can be provided that the setting lever is constructed as a yoke with counter-detent for detenting at the detent tongue in the housing closed setting. In that case it is possible to provide with the same detent element a carrier not only for the squeeze hose bend, but also for the setting lever or setting yoke.

Further advantages, details and features of the invention are evident from the following description as well as on the basis of the drawing, in which:
- Figs. 1 to 4: show, in perspective illustration, different positions of the pump head of the peristaltic pump from the unlocked setting up to the squeeze hose withdrawal setting and
- Fig. 5 and 6: show a cross-section through the housing of the pump head in unlocked and locking setting.

Of the peristaltic pump according to the invention, there is illustrated in the figures the pump head; the corresponding drives are not further depicted here.

The pump head 1 comprises a base element 2 with an integrally formed end wall 3 and two side walls 4 which are in turn integrally formed therewith, as well as a pump housing cover 6 which is displaceable by way of a pivot lever, and which in turn has integrally formed side wall surfaces 7 parallel to the side walls 4 of the base element 2. The side wall surfaces 7 of the pump housing cover 6 have, at the lower outer edge thereof, integrally formed slide rails 8 which are displaceably guided in corresponding counter rails or grooves in the region of transition from the base of the side walls 4.

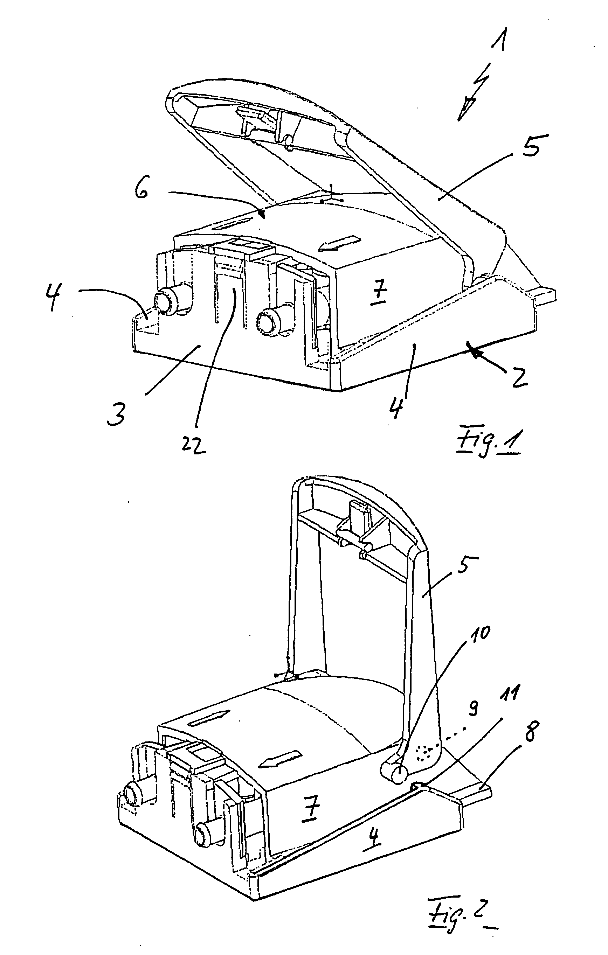

As evident particularly from Fig. 2, the pivot lever 5 is pivotably mounted at the pump housing cover 6 by way of inwardly pointing hinge pins 9, wherein outwardly pointing pivot cams 10 can bear against support detents 11 which are integrally formed at the side walls 4 of the base element 2 to point inwardly.

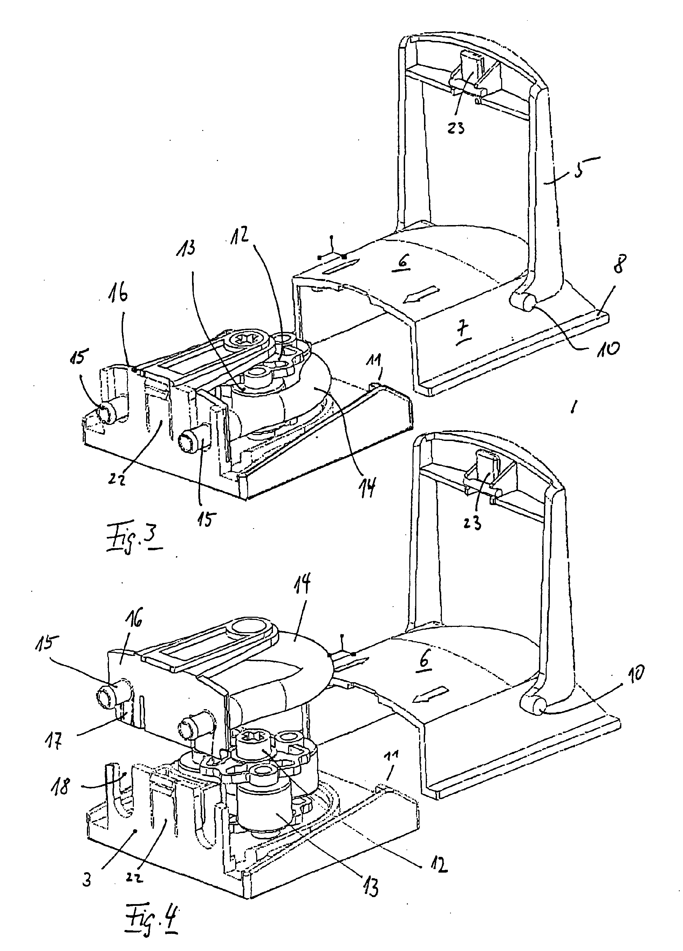

A rotating squeeze roller carrier 12 (Fig. 4) with three squeeze rollers 13, which can be brought into rotation by a drive, which is not illustrated in more detail, is detented in the base element 2. As illustrated in Fig. 3 and 4, a squeeze hose bend 14 fixed by coupling projections 15 in a squeeze hose carrier 16 which can be detented with or released from the end wall 3 by way of sliding guides, wherein the coupling projections 15 project outwardly beyond the entire housing.

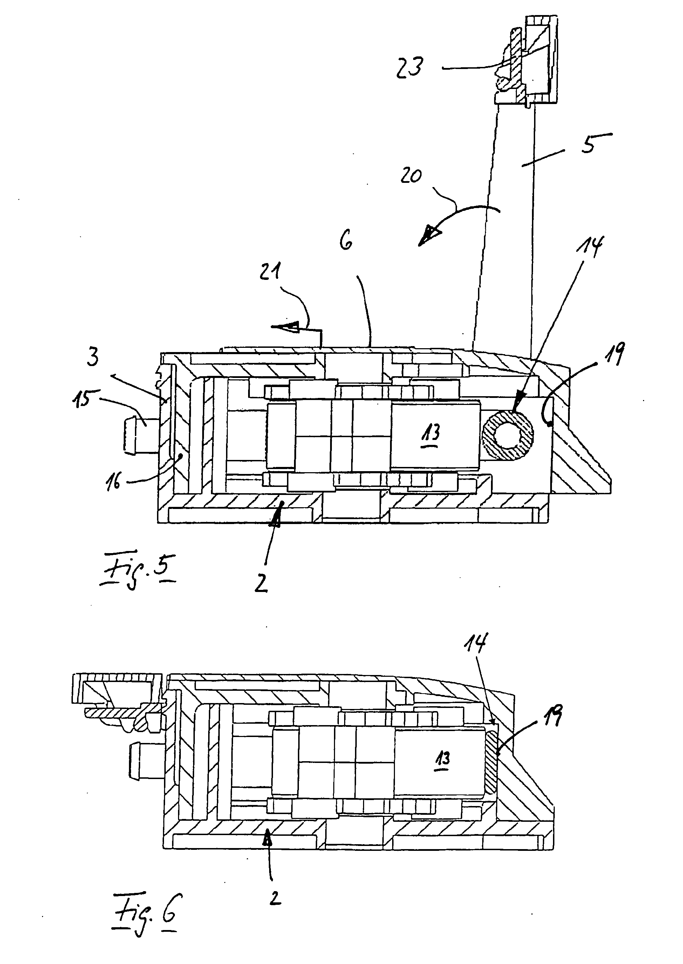

At this point it is to be noted that the squeeze hose bend 14 is fixable also as an individual element by way of alternatively provided slots 17 in the carrier squeeze hose carrier 16 or directly in slots 18 in the end wall 3, depending on the respective construction. The pump housing cover 6 has, in the interior, a curved contact wall 19 for the squeeze hose bend 14, wherein this curved contact wall 19 presses the squeeze hose bend 14 against the squeeze rollers 13 by way of pivotation in accordance with arrow 20 in Fig. 5, as is evident from Fig. 6, which shows the pump head 1 in closed setting. The pump housing cover 6 is displaced in accordance with arrow 21 from the position illustrated in Fig. 5 to the closed position illustrated in Fig. 6,

The end wall 3 is furnished with a detent tongue 22 which co-operates with a counter detent 23 at the pivot lever 5 and detents all elements together in the closed setting.

It can be seen that an exchange of the squeeze hose bend 14 subjected to wear is extremely simple:

From the closed setting illustrated in Fig. 6, the pivot lever 5 is pivoted upwardly, whereby solely through the resilience of the squeeze hose bend 14, which bears against the curved contact wall 19, the pump housing cover 6 is displaced to the right in the figures, at least into the slightly open position illustrated in Fig. 1. If the pivot lever 5 is now pivoted somewhat further and releases the support detent 11, the pump housing cover 6 can be withdrawn from the base element 2 by way of the slide rails 8, this position being illustrated in Fig. 3. At the same time the squeeze hose bend 14 is freed so that the squeeze hose carrier 16 together with the squeeze hose bend 14 can be withdrawn upwardly, as is depicted in Fig. 4. After placing a new squeeze hose bend 14 around the squeeze rollers 13 there takes place a pushing together of the elements and finally locking in the working position illustrated in Fig. 6.

## Claims

1. Peristaltic pump, comprising
a pump head (1) with a base element (2),
a rotating squeeze roller carrier (12) carrying squeeze rollers (13), the rotating squeeze roller carrier (12) being detented in the base element (2),
a squeeze hose bend (14) partly looping around the squeeze rollers (13),
a curved contact wall (19) for the squeeze hose bend (14), and
a pivot lever (5),
wherein in a looking setting of the pump head (1) the curved contact wall (19) being opposite to the effective surface of the squeeze rollers (13) presses the squeeze hose bend (14) against the squeeze rollers (13),
wherein the curved contact wall (19) is movable by way of the pivot lever (5) from an unlocked setting of the pump head (1), which enables fitting with the squeeze hose bend (14), to said locking setting of the pump head (1) pressing the squeeze hose bend (14) against the squeeze rollers (13),
**characterized in that**
the curved contact wall (19) forms a part of a pump housing cover (6) on which the pivot lever (5) is positioned to be outwardly pivotable for displacement of the pump housing cover (6),
the base element (2) has an integrally formed end wall (3),
the squeeze hose bend (14) is provided on a squeeze hose carrier (16), which is positioned at the end wall (3) of the base element (2) in the locking setting of the pump head (1), and the squeeze hose carrier (16) together with the squeeze hose bend (14) is freed to be withdrawn from the pump head (1) in the unlocked setting thereof.

2. Peristaltic pump according to claim 1, **characterized in that**
the base element (2) is provided with counter rails or grooves and the pump housing cover (6) is provided with integrally formed slide rails (8) which are displaceably guided in the counter rails or grooves on the base element (2).

3. Peristaltic pump according to any one of the preceding claims, **characterized in that**
the pivot lever (5) is provided with pivot cams (10) and the base element (2) is provided with support detents (11) for receiving the pivot cams (10) of the pivot lever (5).

4. Peristaltic pump according to any one of the preceding claims, **characterized in that**
the squeeze hose bend (14) is fixed by coupling projections (15) in the squeeze hose carrier (16).

5. Peristaltic pump according to any one of the preceding claims, **characterized in that**
the end wall (3) has two substantially U-shaped recesses (18) for passage of the squeeze hose bend (14).

6. Peristaltic pump according to any one of the preceding claims, **characterized in that**
the end wall (3) has a detent tongue (22).

7. Peristaltic pump according to claim 6, **characterized in that**
the pivot lever (5) is constructed as a yoke with a counter detent (23) for detenting on the detent tongue (22) in the locking setting of the pump head (1).

## Patentansprüche

1. Schlauchquetschpumpe, umfassend:
einen Pumpenkopf (1) mit einem Basiselement (2),
einen Quetschrollenträger (12), der Quetschrollen (13) trägt, wobei der rotierende Quetschrollenträger (12) in dem Basiselement (2) verrastet ist,
einen Quetschschlauchbogen (14), der die Quetschrollen (13) teilweise umschlingt,
eine gekrümmte Anlagewand (19) für den Quetschschlauchbogen (14), und
einen Schwenkhebel (5),
wobei in einer Verriegelungsstellung des Pumpenkopfs (1) die gekrümmte Anlagewand (19), die der Wirkfläche der Quetschrollen (13) gegenüberliegt, den Quetschschlauchbogen (14) gegen die Quetschrollen (13) drückt,
wobei die gekrümmte Anlagewand (19) durch den Schwenkhebel (5) aus einer entriegelten Stellung des Pumpenkopfs (1), die Versehen mit dem Quetschschlauchbogen (14) ermöglicht, in die Verriegelungsstellung des Pumpenkopfs (1), in der der Quetschschlauchbogen (14) gegen die Quetschrollen (13) gedrückt wird, bewegt werden kann,
**dadurch gekennzeichnet, dass** die gekrümmte Anlagewand (19) einen Teil eines Pumpengehäusedeckels (6) bildet, an dem der Schwenkhebel (5) so positioniert ist, dass er zur Verschiebung des Pumpengehäusedeckels (6) nach außen geschwenkt werden kann,
das Basiselement (2) eine integral geformte Endwand (3) aufweist,
der Quetschschlauchbogen (14) auf einem Quetschschlauchträger (16) vorgesehen ist, der in der Verriegelungsstellung des Pumpenkopfs (1) an der Endwand (3) des Basiselements (2) positioniert ist, und der Quetschschlauchträger (16) zusammen mit dem Quetschschlauchbogen (14) in der entriegelten Stellung davon zum Zurückziehen von dem Pumpenkopf (1) freigegeben ist.

2. Schlauchquetschpumpe nach Anspruch 1, **dadurch gekennzeichnet, dass**
das Basiselement (2) mit Gegenschienen oder Nuten versehen ist und der Pumpengehäusedeckel (6) mit integral geformten Gleitschienen (8) versehen ist, die in den Gegenschienen oder Nuten am Basiselement (2) verschiebbar geführt werden.

3. Schlauchquetschpumpe nach einem der vorhergehenden Ansprüche, **dadurch gekennzeichnet, dass**
der Schwenkhebel (5) mit Schwenknocken (10) versehen ist und das Basiselement (2) mit Anlagerasten (11) zur Aufnahme der Schwenknocken (10) des Schwenkhebels (5) versehen ist.

4. Schlauchquetschpumpe nach einem der vorhergehenden Ansprüche, **dadurch gekennzeichnet, dass**
der Quetschschlauchbogen (14) durch Kupplungsvorsprünge (15) in dem Quetschschlauchträger (16) befestigt ist.

5. Schlauchquetschpumpe nach einem der vorhergehenden Ansprüche, **dadurch gekennzeichnet, dass**
die Endwand (3) zwei im Wesentlichen U-förmige Aussparungen (18) zum Durchtritt des Quetschschlauchbogens (14) aufweist.

6. Schlauchquetschpumpe nach einem der vorhergehenden Ansprüche, **dadurch gekennzeichnet, dass**
die Endwand (3) eine Rastzunge (22) aufweist.

7. Schlauchquetschpumpe nach einem der vorhergehenden Ansprüche, **dadurch gekennzeichnet, dass**
der Schwenkhebel (5) als ein Joch mit einer Gegenraste (23) zur Verrastung an der Rastzunge (22) in der Verriegelungsstellung des Pumpenkopfs (1) ausgeführt ist.

## Revendications

1. Pompe péristaltique, comprenant :
une tête de pompe (1) avec un élément de base (2),
un support de rouleaux de compression rotatif (12) portant des rouleaux de compression (13), le support de rouleaux de compression rotatif (12) étant encliqueté dans l'élément de base (2),
un coude de tuyau de compression (14) s'enroulant en partie autour des rouleaux de compression (13),
une paroi de contact courbe (19) pour le coude de tuyau de compression (14), et
un levier pivotant (5),
la paroi de contact courbe (19) opposée à la surface efficace des rouleaux de compression (13),
dans une configuration de verrouillage de la tête de pompe (1), pressant le coude de tuyau de compression (14) contre les rouleaux de compression (13),
la paroi de contact courbe (19) pouvant être déplacée au moyen du levier pivotant (5) d'une configuration déverrouillée de la tête de pompe (1), qui permet un ajustement avec le coude de tuyau de compression (14), dans ladite configuration de verrouillage de la tête de pompe (1) pressant le coude de tuyau de compression (14) contre les rouleaux de compression (13),
**caractérisée en ce que**
la paroi de contact courbe (19) fait partie d'un recouvrement de carter de pompe (6) sur lequel le levier pivotant (5) est positionné de manière à pouvoir pivoter vers l'extérieur pour déplacer le recouvrement de carter de pompe (6),
l'élément de base (2) a une paroi d'extrémité (3) formée intégralement,
le coude de tuyau de compression (14) est pourvu d'un support de tuyau de compression (16) qui est positionné au niveau de la paroi d'extrémité (3) de l'élément de base (2) dans la configuration de verrouillage de la tête de pompe (1), et le support de tuyau de compression (16),
conjointement avec le coude de tuyau de compression (14), est libéré de manière à pouvoir être retiré de la tête de pompe (1) dans sa configuration déverrouillée.

2. Pompe péristaltique selon la revendication 1, **caractérisée en ce que**
l'élément de base (2) est pourvu de rails ou de gorges conjugués et le recouvrement de carter de pompe (6) est pourvu de rails de glissement formés intégralement (8) qui sont guidés de manière déplaçable dans les rails ou gorges conjugués sur l'élément de base (2).

3. Pompe péristaltique selon l'une quelconque des revendications précédentes,
**caractérisée en ce que**
le levier pivotant (5) est pourvu de cames de pivotement (10) et l'élément de base (2) est pourvu d'encliquetages de support (11) pour recevoir les cames de pivotement (10) du levier pivotant (5).

4. Pompe péristaltique selon l'une quelconque des revendications précédentes,
**caractérisée en ce que**
le coude de tuyau de compression (14) est fixé par des saillies d'accouplement (15) dans le support de tuyau de compression (16).

5. Pompe péristaltique selon l'une quelconque des revendications précédentes,
**caractérisée en ce que**
la paroi d'extrémité (3) a deux retraits (18) substantiellement en forme de U pour le passage du coude de tuyau de compression (14).

6. Pompe péristaltique selon l'une quelconque des revendications précédentes,
**caractérisée en ce que**
la paroi d'extrémité (3) a une langue d'encliquetage (22).

7. Pompe péristaltique selon la revendication 6, **caractérisée en ce que**
le levier de pivotement (5) est construit sous forme d'étrier avec un cliquet conjugué (23) pour s'encliqueter sur la langue d'encliquetage (22) dans la configuration de verrouillage de la tête de pompe (1).
